Europäisches Patentamt

European Patent Office

Office européen des brevets

(19)

(11) Numéro de publication: **0 370 856 B2**

(12) **NOUVEAU FASCICULE DE BREVET EUROPÉEN**

(45) Date de publication de nouveau fascicule du brevet: **30.08.95**

(51) Int. Cl.⁶: **A61K 7/02**

(21) Numéro de dépôt: **89403098.0**

(22) Date de dépôt: **09.11.89**

(54) **Démaquillant pour les yeux à deux phases distinctes.**

(30) Priorité: **09.11.88 FR 8814641**

(43) Date de publication de la demande:
**30.05.90 Bulletin 90/22**

(45) Mention de la délivrance du brevet:
**29.07.92 Bulletin 92/31**

(45) Mention de la décision
concernant l'opposition:
**30.08.95 Bulletin 95/35**

(84) Etats contractants désignés:
**AT BE CH DE ES FR GB GR IT LI NL SE**

(56) Documents cités:
**EP-A- 0 141 732**    **DE-A- 1 617 808**
**DE-A- 2 905 257**    **DE-A- 3 110 258**
**DE-C- 3 627 313**    **FR-A- 2 101 710**
**FR-A- 2 254 636**    **LU-A- 51 328**

**PATENT ABSTRACTS OF JAPAN, vol. 12, no. 237 (C-509)[3084], 6 juillet 1988**

**K. Schrader, Grundlagen der Rezepturen der Kosmetika (1979), S. 238**

(73) Titulaire: **L'OREAL**
**14, rue Royale**
**F-75008 Paris (FR)**

(72) Inventeur: **Zabotto-Arribau, Arlette**
**24, Rue Sarrette**
**F-75014 Paris (FR)**
Inventeur: **Contamin, Jean-Claude**
**14 Avenue du Château**
**F-91420 Morangis (FR)**
Inventeur: **Plaisant, Nathalie**
**13, Rue du Guesclin**
**F-94240 L'Hay les Roses (FR)**

(74) Mandataire: **Stalla-Bourdillon, Bernard et al**
**CABINET NONY & CIE**
**29, rue Cambacérès**
**F-75008 Paris (FR)**

EP 0 370 856 B2

**Description**

La présente invention a pour objet un démaquillant pour les yeux constitué de deux phases distinctes, une phase aqueuse et une phase huileuse.

Le maquillage des yeux consiste en l'application sur les paupières et les cils d'une composition contenant des pigments colorés, celle-ci étant ou non à base d'une huile; pour les paupières il s'agit d'un fard et pour les cils d'un mascara.

Les produits de maquillage à base d'huile sont généralement désignés sous la dénomination de "waterproof" (WP) alors que ceux n'en contenant pas sont désignés sous la dénomination de "non-waterproof" (NWP).

L'élimination du premier type de maquillage (WP) est généralement réalisée à l'aide d'un démaquillant à base d'une huile en particulier d'huile de vaseline éventuellement mélangée à des esters gras. Ces démaquillants sont bien entendu susceptibles d'éliminer les maquillages du deuxième type (NWP), mais ils présentent de tels désagréments à l'application que lorsqu'ils ne sont pas indispensables, on préfère employer une lotion démaquillante classique.

Ces lotions qui contiennent en solution aqueuse un agent tensio-actif permettent en général une bonne élimination des maquillages du type NWP mais présentent l'inconvénient d'assécher la peau par élimination de son film gras naturel.

Il résulte de ce qui précède qu'en fonction du type de maquillage utilisé, il convient d'employer un démaquillant approprié. Toutefois quel que soit le type de démaquillant employé, il est particulièrement difficile de concilier une bonne élimination du maquillage tout en conservant de bonnes caractéristiques cosmétiques telles que l'agrément et le confort pendant et après l'application.

La présente invention apporte une solution au problème du démaquillage des yeux en proposant, pour la première fois, une composition susceptible d'éliminer indifféremment les maquillages du type WP et NWP d'une manière particulièrement efficace et dans des conditions très satisfaisantes quant aux propriétés cosmétiques.

En effet les compositions de démaquillage selon l'invention ne provoquent pendant leur application aucune sensation de tiraillement ou d'irritation et confèrent à la peau de la fraicheur ainsi que de la douceur par formation d'un film gras reconstituant le film gras naturel de la peau.

La présente invention a pour objet une composition cosmétique pour les yeux, non moussante et utilisable pour l'élimination des maquillages du type "waterproof" et "non waterproof", constituée de deux phases distinctes, une phase inférieure ou phase aqueuse contenant au moins un agent tensio-actif du type anionique, non-ionique ou amphotère et une phase supérieure ou phase huileuse comprenant au moins une huile cosmétique dont au moins 8% est une huile de silicone le rapport pondéral entre la phase inférieure et la phase supérieure étant compris entre 30/70 et 60/40 et la concentration en agent tensio-actif étant comprise entre 0,1 et 3% en poids.

Les études comparatives qui ont été réalisées et qui seront rapportées ci-après, permettent de mettre en évidence un effet de synergie entre les deux phases en ce qui concerne non seulement les propriétés démaquillantes mais également les propriétés cosmétiques.

La phase aqueuse peut être constituée par de l'eau déminéralisée stérile ou par une eau florale telle que de l'eau de rose, de l'eau de bleuet, de l'eau de camomille ou de l'eau de tilleul.

L'agent tensio-actif qui peut être du type anionique, non-ionique ou amphotère mais de préférence du type non-ionique est de préférence présent dans la phase aqueuse en une proportion comprise entre 0,1 et 1,5% (de matière active) en poids par rapport au poids total de la composition démaquillante.

Parmi les agents tensio-actifs non-ioniques, ceux particulièrement préférés sont:
- les esters gras de sorbitol polyoxyéthylénés tels que le produit vendu sous la dénomination de "TWEEN 20" par la Société ATLAS,
- les alcools gras polyoxyéthylénés tels que le produit vendu sous la dénomination de "REMCOPAL 21912 AL" par la Société GERLAND,
- les alkylphénols polyoxyéthylénés tels que le produit vendu sous la dénomination de "TRITON X 100" par la Société ROHM-HAAS, et
- les condensats d'oxyde d'éthylène et d'oxyde de propylène tels que ceux vendus sous les dénominations de "SYNPERONIC PE" par la Société ICI et en particulier ceux référencés L 31, L 64, F 38, F 88, L 92, P 103, F 108 et F 127.

Parmi les agents tensio-actifs anioniques on peut notamment citer:
- les alkyléthers sulfates tels que le produit vendu sous la dénomination de "TEXAPON ASV" par la Société HENKEL,

2

- les alkylsulfoacétates tels que le produit vendu sous la dénomination de "LATHANOL LAL" par la Société STEPAN,
- les sulfosuccinates d'alkyle tels que le produit vendu sous la dénomination de "SODIUM DIOCTYL SULFOSUCCINATE" par la Société RHONE POULENC,
- les alkylamido sulfosuccinates tels que le produit vendu sous la dénomination de "REWODERM S 1333" par la Société REWO,
- les alkylamido polypeptides tels que le produit vendu sous la dénomination de "LAMEPON S" par la Société GRUNAU, et
- les acylsarcosinates tels que le produit vendu sous la dénomination de "ORAMIX L 30" par la Société SEPPIC.

Parmi les tensio-actifs amphotères, on peut notamment citer:
- les alkylamidopropyl diméthylbétaïnes tels que le produit vendu sous la dénomination de "TEGO BETAINE L 7" par la Société GOLDSCHMIDT,
- les alkylamidobétaïnes tels que le produit vendu sous la dénomination de "INCRONAM 30" par la Société CRODA,
- les dérivés d'imidazoline tels que le produit vendu sous la dénomination de "CHIMEXANE HD" par la Société CHIMEX, et
- les N-alkyl $\beta$-imino-dipropionates tels que le produit vendu sous la dénomination de "MONATERIC ISA 35" par la Société MONA.

La phase huileuse de la composition démaquillante selon l'invention est constituée d'un mélange d'huiles, celles-ci pouvant être des huiles minérales, végétales ou synthétiques ou encore des huiles de silicone.

Parmi les huiles minérales pouvant constituer la phase huileuse, on peut notamment citer l'huile de vaseline et les hydrocarbures aliphatiques supérieurs tels que par exemple l'isohexadecane ; parmi les huiles végétales, l'huile de jojoba, ainsi que l'huile de carthame; parmi les huiles de silicone, le cyclopentadiméthylsiloxane vendu sous la dénomination de "VOLATIL SILICONE 7158" par la Société UNION CARBIDE et parmi les produits de synthèse, les palmitates d'alkyle ayant de 2 à 10 atomes de carbone tels que le palmitate d'isopropyle, ou le palmitate d'éthyl-2 hexyle et les adipates d'alkyle ayant de 2 à 10 atomes de carbone tels que l'adipate de di-éthyl-2 hexyle.

Selon une forme de réalisation particulière de l'invention, la phase huileuse contient au moins un palmitate d'alkyle ayant de 2 à 10 atomes de carbone en une proportion d'au moins 8% et de préférence comprise entre 10 et 30% par rapport au poids total de la composition démaquillante.

Selon une forme préférée de l'invention l'huile de silicone est présente en une proportion comprise entre 15 et 50% par rapport au poids total de la composition démaquillante.

La composition démaquillante selon l'invention peut également contenir des adjuvants cosmétiques conventionnels qui se trouveront dans l'une ou l'autre phase selon leur nature hydrophile ou lipophile tels que par exemple des parfums, des agents conservateurs, des colorants, des agents adoucissants, un tampon, des humectants et éventuellement un électrolyte tel que le chlorure de sodium pour apporter une isotonicité dans la phase aqueuse.

Parmi les agents humectants, on peut notamment mentionner, l'hexylèneglycol et le polyéthylèneglycol 600, ceux-ci étant présents à une concentration inférieure ou égale à 5 % et de préférence comprise entre 0,05 et 2%.

Parmi les agents adoucissants, on peut en particulier citer l'allantoïne, et certains extraits de plantes.

On va maintenant donner à titre d'illustration plusieurs exemples de compositions démaquillantes pour les yeux selon l'invention:

EXEMPLE 1

Une composition démaquillante biphase, selon l'invention, est obtenue en conditionnant dans un flacon 50% d'une phase huileuse (A) et 50% d'une phase aqueuse (B) contenant les ingrédients suivants:

| A. Phase huileuse | % |
|---|---|
| - Palmitate d'éthyl-2 hexyle | 20 |
| - Adipate de di-éthyl-2 hexyle | 20 |
| - Cyclopentadiméthylsiloxane | 60 |

3

| B. Phase aqueuse | % |
|---|---|
| - Laurate de sorbitan oxyéthyléné à 20 moles d'oxyde d'éthylène | 0,5 |
| - Hexylène glycol | 0,5 |
| - Phosphate monopotassique | 0,1 |
| - Phosphate dipotassique | 0,3 |
| - Chlorure de sodium | 0,9 |
| - Parfums | qs |
| - Colorants | qs |
| - Conservateurs | qs |
| - Eau déminéralisée     qsp | 100 |

EXEMPLE 2

Une composition démaquillante biphase, selon l'invention, est obtenue en conditionnant dans un flacon 50% d'une phase huileuse (A) et 50% d'une phase aqueuse (B) contenant les ingrédients suivants:

| A. Phase huileuse | % |
|---|---|
| - Palmitate d'isopropyle | 40 |
| - Huile de jojoba | 2 |
| - Cyclopentadiméthylsiloxane | 58 |

| B. Phase aqueuse | % |
|---|---|
| - Condensat d'oxyde d'éthylène et d'oxyde de propylène vendu par la Société ICI sous la dénomination "SYNPERONIC PE/F38" | 2 |
| - Propylène glycol | 0,4 |
| - Triéthanolamine | 0,08 |
| - Chlorure de sodium | 0,8 |
| - Parfums | qs |
| - Conservateurs | qs |
| - Colorants | qs |
| - Eau déminéralisée     qsp | 100 |

EXEMPLE 3

Une composition démaquillante biphase, selon l'invention, est obtenue en conditionnant dans un flacon 70% d'une phase huileuse (A) et 30% d'une phase aqueuse (B) contenant les ingrédients suivants:

| A. Phase huileuse | % |
|---|---|
| - Huile de vaseline | 25 |
| - Palmitate d'éthyl-2 hexyle | 25 |
| - Cyclopentadiméthylsiloxane | 50 |

| B. Phase aqueuse | % |
|---|---|
| - Hydroxyéthyl-1 lauryl-2 carboxyméthyl-3 imidazolinium bétaïne | 2 |
| - Allantoïne | 0,15 |
| - Triéthanolamine | 0,04 |
| - Parfums | qs |
| - Conservateurs | qs |
| - Colorants | qs |
| - Eau déminéralisée    qsp | 100 |

EXEMPLE 4

Une composition démaquillante biphase, selon l'invention est obtenue en additionnant dans un flacon 50% de la phase huileuse (A) et 50 % de la phase aqueuse (B) contenant les ingrédients suivants :

| A. Phase huileuse | % |
|---|---|
| - Cyclopentadimethylsiloxane | 59,75 |
| - Isohexadecane | 40,00 |
| - Alcool benzylique | 0,25 |

| B. Phase aqueuse | |
|---|---|
| - Condensat d'oxyde d'éthylène et d'oxyde de propylène vendu sous la dénomination commerciale de "SYMPERONIC PE/F38 par la Société ICI | 0,5 |
| - Phosphate monopotassique | 0,1 |
| - Phosphate dipotassique | 0,3 |
| - Chlorure de sodium | 0,9 |
| - Hexylène glycol | 0,5 |
| - Conservateurs | qs |
| - Parfum | qs |
| - Eau    qsp | 100 |

ETUDES COMPARATIVES

Afin de mettre en évidence les qualités démaquillantes pour les yeux et les bonnes propriétés cosmétiques des compositions selon l'invention on a comparé les démaquillants suivants:

DEMAQUILLANT A : Ce démaquillant correspond à celui de l'exemple 1 tel que décrit ci-dessus.
DEMAQUILLANT B : lotion démaquillante aqueuse ayant la composition suivante:

| | % |
|---|---|
| - Laurate de sorbitan oxyéthyléné à 20 moles d'oxyde d'éthylène | 0,5 |
| - Hexylène glycol | 0,5 |
| - Phosphate monopotassique | 0,1 |
| - Phosphate dipotassique | 0,3 |
| - Chlorure de sodium | 0,9 |
| - Parfums | qs |
| - Conservateurs | qs |
| - Colorants | qs |
| - Eau déminéralisée    qsp | 100 |

Cette composition correspond à la phase aqueuse du démaquillant de l'exemple 1 ci-dessus.

DEMAQUILLANT C : Huile démaquillante ayant la composition suivante:

|  | % |
|---|---|
| - Palmitate d'éthyl-2 hexyle | 20 |
| - Adipate de di-éthyl-2 hexyle | 20 |
| - Cyclopentadiméthylsiloxane | 60 |

Cette huile correspond à la phase huileuse du démaquillant de l'example 1 ci-dessus.

DEMAQUILLANT D : Composition démaquillante biphase constituée de:

50% d'une phase huileuse ayant la composition suivante:

|  | % |
|---|---|
| - Palmitate d'éthyl-2 hexyle | 20 |
| - Adipate de di-éthyl-2 hexyle | 20 |
| - Cyclopentadiméthylsiloxane et de 50% d'eau. | 60 |

La phase huileuse de cette composition correspond à celle de l'exemple 1 ci-dessus.

On a formé 5 groupes de 17 femmes chacun. A chaque groupe il a été remis:

1er groupe : un mascara NWP et un flacon des démaquillants A et B,

2ème groupe: un mascara NWP et un flacon des démaquillants A et C,

3ème groupe: un mascara NWP et un flacon des démaquillants A et D,

4ème groupe: un mascara WP et un flacon des démaquillants A et C,

5ème groupe: un mascara WP et un flacon des démaquillants A et D.

Il a été demandé à chaque femme de se maquiller les cils, à l'aide du mascara remis, pendant une semaine chaque jour et de se démaquiller le soir en utilisant pour l'oeil droit le démaquillant A selon l'invention et pour l'oeil gauche l'autre démaquillant de comparaison.

Après cette période, les impressions des utilisatrices ont été recueillies et sont rapportées dans les deux Tableaux (I) et (II) suivants:

EP 0 370 856 B2

'TABLEAU I

| MASCARA NWP | | | COMPOSITIONS DEMAQUILLANTES | | | |
|---|---|---|---|---|---|---|
| | | | A * | B | C | D |
| Qualités du démaquillage | BON | | 16 ' | 3 | 13 | 8 |
| | MAUVAIS | | 1 | 14 | 4 | 9 |
| Propriétés cosmétiques | Fraîcheur | bonne | 13 | 17 · | 3 | 8 |
| | | insuffisante | 4 | 0 | 14 | 9 |
| | Douceur | bonne | 17 | 17 | 9 | 5 |
| | | mauvaise | 0 | 0 | 8 | 12 |
| | Aspect final de la peau | film gras naturel | 14 | 0 | 17 | 14 |
| | | rien à signaler | 3 | 17 | 0 | 3 |
| Inconvénients | Picotement | | 0 | 0 | 0 | 0 |
| | Gêne | | 0 | 0 | 3 | 3 |
| | Inconfort | | 2,5 | 0 | 6 | 6 |
| | Rien à signaler | | 14,5 | 17 | 8 | 8 |

* Valeur moyenne des trois groupes ayant utilisé un mascara NWP.

TABLEAU II

| MASCARA WP | | | COMPOSITIONS DEMAQUILLANTES | | |
|---|---|---|---|---|---|
| | | | A * | C | D |
| Qualités du démaquillage | BON | | 15 | 15 | 2 |
| | MAUVAIS | | 2 | 2 | 15 |
| Propriétés cosmétiques | Fraîcheur | Bonne | 13 | 2 | 8 |
| | | Insuffisante | 4 | 15 | 9 |
| | Douceur | Bonne | 17 | 8 | 5 |
| | | Mauvaise | 0 | 9 | 12 |
| | Aspect final de la peau | Film gras naturel | 14 | 17 | 14 |
| | | Rien à signaler | 3 | 0 | 3 |
| Inconvénients | Picotement | | 0 | 0 | 0 |
| | Gêne | | 1 | 3 | 4 |
| | Inconfort | | 3 | 6 | 6 |
| | Rien à signaler | | 13 | 8 | 7 |

\* Valeur moyenne des deux groupes ayant utilisé un mascara WP.

Comme on peut le constater d'après le Tableau (I), le démaquillant A selon l'invention associe à la fois de bonnes qualités démaquillantes et de bonnes propriétés cosmétiques à l'égard d'un mascara NWP, ce qui n'est pas le cas des autres compositions démaquillantes. La comparaison avec la composition démaquillante D met en évidence l'importance de la présence d'un agent tensio-actif dans la phase aqueuse du démaquillant selon l'invention.

Le Tableau II montre que si les qualités du démaquillage du démaquillant A selon l'invention à l'égard d'un mascara WP sont comparables à celle du démaquillant C, les propriétés cosmétiques sont par contre nettement supérieures notamment quant à la fraîcheur et à la douceur apportées par la phase aqueuse contenant un agent tensio-actif du démaquillant selon l'invention, durant et après l'utilisation.

Les marques commerciales deposées sont reconnues comme telles.

**Revendications**

1. Composition cosmétique démaquillante pour les yeux, non moussante et utilisable pour l'élimination des maquillages du type "waterproof" et "non waterproof", caractérisée par le fait qu'elle est constituée de deux phases distinctes, une phase inférieure ou phase aqueuse contenant au moins un agent tensioactif du type anionique, non ionique ou amphotère, et une phase supérieure ou phase huileuse comprenant au moins une huile cosmétique dont au moins 8 % est une huile de silicone, le rapport pondéral entre la phase inférieure et la phase supérieure étant compris entre 30/70 et 60/40 et la concentration en agent tensioactif étant comprise entre 0,1 et 3 % en poids par rapport au poids total de la composition.

2. Composition selon la revendication 1, caractérisée par le fait que la phase aqueuse est constituée par de l'eau déminéralisée stérile, de l'eau de rose, de l'eau de bleuet, de l'eau de camomille ou de l'eau de tilleul.

3. Composition selon l'une quelconque des revendications 1 et 2, caractérisée par le fait que l'agent tensioactif est du type non-ionique.

4. Composition selon l'une quelconque des revendications 1 à 3, caractérisée par le fait que l'agent tensioactif est présent en une concentration comprise entre 0,1 et 1,5 % en poids par rapport au poids total de la composition.

5. Composition selon la revendication 1, caractérisée par le fait que la phase huileuse contient au moins une huile prise dans le groupe constitué par : l'huile de vaseline, l'isohexadecane, l'huile de jojoba, l'huile de carthame, une huile de silicone ou une huile synthétique.

6. Composition selon la revendication 5, caractérisée par le fait que l'huile synthétique est un palmitate d'alkyle ou un adipate d'alkyle, le radical alkyle ayant de 2 à 10 atomes de carbone.

7. Composition selon l'une quelconque des revendications précédentes, caractérisée par le fait que la phase huileuse contient au moins un palmitate d'alkyle ayant de 2 à 10 atomes de carbone en une proportion d'au moins 8 % en poids par rapport au poids total de la composition.

8. Composition selon la revendication 7, caractérisée par le fait que la phase huileuse contient de 10 à 30 % d'un palmitate d'alkyle ayant de 2 à 10 atomes de carbone, par rapport au poids total de la compositions.

9. Composition selon l'une quelconque des revendications 7 et 8, caractérisée par le fait que le palmitate d'alkyle est le palmitate d'isopropyle ou le palmitate d'éthyl-2 hexyle.

10. Composition selon l'une quelconque des revendications précédentes, caractérisée par le fait que l'huile de silicone est présente en une proportion comprise entre 15 et 50 % par rapport au poids total de la composition démaquillante.

11. Composition selon l'une quelconque des revendications précédentes, caractérisée par le fait qu'elle contient en outre dans l'une ou l'autre phase au moins un adjuvant cosmétique conventionnel pris dans le groupe constitué par : un parfum, un agent conservateur, un colorant, un agent adoucissant, un tampon, un humectant ou un électrolyte tel que le chlorure de sodium.

**Claims**

1. Non-foaming makeup-removal cosmetic composition for the eyes, usable for the removal of makeup of the waterproof and non-waterproof type, characterised in that it consists of two distinct phases, a lower phase or aqueous phase containing at least one surfactant of the anionic, non-ionic or amphoteric type, and an upper phase or oily phase comprising at least one cosmetic oil of which at least 8% is a silicone oil, the weight ratio between the lower phase and the upper phase being between 30:70 and 60:40 and the concentration of surfactant being between 0.1 and 3 % by weight relative to the total weight of the composition.

**2.** Composition according to Claim 1, characterised in that the aqueous phase consists of sterile demineralised water, of rose water, of cornflower water, of chamomile water or of lime-flower water.

**3.** Composition according to either of Claims 1 and 2, characterised in that the surfactant is of the non-ionic type.

**4.** Composition according to any one of Claims 1 to 3, characterised in that the surfactant is present in a concentration of between 0.1 and 1.5 % by weight relative to the total weight of the composition.

**5.** Composition according to Claim 1, characterised in that the oily phase contains at least one oil selected from the group consisting of: liquid paraffin, isohexadecane, jojoba oil, safflower oil, a silicone oil or a synthetic oil.

**6.** Composition according to Claim 5, characterised in that the synthetic oil is an alkyl palmitate or an alkyl adipate, the alkyl radical having from 2 to 10 carbon atoms.

**7.** Composition according to any one of the preceding claims, characterised in that the oily phase contains at least one alkyl palmitate, the alkyl radical having from 2 to 10 carbon atoms, in a proportion of at least 8 % by weight relative to the total weight of the composition.

**8.** Composition according to Claim 7, characterised in that the oily phase contains from 10 to 30 % of an alkyl palmitate, the alkyl radical having from 2 to 10 carbon atoms, relative to the total weight of the composition.

**9.** Composition according to either of Claims 7 and 8, characterised in that the alkyl palmitate is isopropyl palmitate or 2-ethylhexyl palmitate.

**10.** Composition according to any one of the preceding claims, characterised in that the silicone oil is present in a proportion of between 15 and 50 % relative to the total weight of the makeup-removal composition.

**11.** Composition according to any one of the preceding claims, characterised in that it contains, in addition, in either phase, at least one conventional cosmetic adjuvant selected from the group consisting of: a fragrance, a preservative, a colouring, an emollient, a buffer, a humectant or an electrolyte such as sodium chloride.

**Patentansprüche**

**1.** Nichtschäumendes kosmetisches Mittel für die Augen, zum Entfernen von wasserfester und nicht-wasserfester Schminke, dadurch gekennzeichnet, daß es aus zwei verschiedenen Phasen besteht, einer unteren oder wäßrigen Phase, welche mindestens ein oberflächenaktives, anionisches, nichtionisches oder amphoteres Mittel enthält, und einer oberen oder öligen Phase, welche mindestens ein kosmetisches Öl mit wenigstens 8% eines Silikonöls umfaßt, wobei das Gewichtsverhältnis zwischen der unteren Phase und der oberen Phase bei 30/70 bis 60/40 liegt und die Konzentration des oberflächenaktiven Mittels 0,1 bis 3 Gew.-%, bezogen auf das Gesamtgewicht des Mittels, beträgt.

**2.** Mittel nach Anspruch 1, dadurch gekennzeichnet, daß die wäßrige Phase aus sterilem, entmineralisiertem Wasser, Rosenwasser, Kornblumenwasser, Kamillenwasser oder Lindenblütenwasser besteht.

**3.** Mittel nach einem der Ansprüche 1 oder 2, dadurch gekennzeichnet, daß das oberflächenaktive Mittel ein nichtionisches Mittel ist.

**4.** Mittel nach einem der Ansprüche 1 bis 3, dadurch gekennzeichnet, daß das oberflächenaktive Mittel in einer Konzentration von 0,1 bis 1,5 Gew.-%, bezogen auf das Gesamtgewicht des Mittels, enthalten ist.

**5.** Mittel nach Anspruch 1, dadurch gekennzeichnet, daß die Ölphase mindestens ein Öl enthält, ausgewählt unter: Vaselineöl, Isohexadekan, Jojobaöl, Safloröl, Silikonöl oder einem synthetischen Öl.

6. Mittel nach Anspruch 5, dadurch gekennzeichnet, daß das synthetische Öl ein Alkylpalmitat oder Alkyladipat ist, wobei der Alkylrest 2 bis 10 Kohlenstoffatome aufweist.

7. Mittel nach einem der vorhergehenden Ansprüche, dadurch gekennzeichnet, daß die Ölphase mindestens ein $C_{2-10}$-Alkylpalmitat in einem Anteil von mindestens 8 Gew.-%, bezogen auf das Gesamtgewicht des Mittels, enthält.

8. Mittel nach Anspruch 7, dadurch gekennzeichnet, daß die Ölphase 10 bis 30% eines $C_{2-10}$-Alkylpalmitats, bezogen auf das Gesamtgewicht des Mittels, enthält.

9. Mittel nach einem der Ansprüche 7 oder 8, dadurch gekennzeichnet, daß das Alkylpalmitat Isopropylpalmitat oder 2-Ethyl-hexyl-palmitat ist.

10. Mittel nach einem der vorhergehenden Ansprüche, dadurch gekennzeichnet, daß die Ölphase mindestens ein Silikonöl in einem Anteil von 15 bis 50%, bezogen auf das Gesamtgewicht des Abschminkmittels, enthält.

11. Mittel nach einem der vorhergehenden Ansprüche, dadurch gekennzeichnet, daß es außerdem in der einen oder anderen Phase mindestens ein herkömmliches kosmetisches Hilfsmittel enthält, welches ausgewählt ist unter einem Parfüm, einem Konservierungsmittel, einem Farbstoff, einem weichmachenden Mittel, einem Puffer, einem Feuchthaltemittel oder einem Elektrolyten, wie Natriumchlorid.